(19) Europäisches Patentamt
European Patent Office
Office européen des brevets

(11) Numéro de publication: **0 060 955**
**B1**

(12) **FASCICULE DE BREVET EUROPEEN**

(45) Date de publication du fascicule du brevet: **08.05.85**

(51) Int. Cl.$^4$: **C 07 D 405/04**, A 01 N 43/54

(21) Numéro de dépôt: **81400473.5**

(22) Date de dépôt: **25.03.81**

(54) **Nouveaux uraciles substitués comportant un groupement 2-tétrahydropyranyle, leur procédé de préparation et les compositions pesticides les renfermant.**

(43) Date de publication de la demande:
**29.09.82 Bulletin 82/39**

(45) Mention de la délivrance du brevet:
**08.05.85 Bulletin 85/19**

(84) Etats contractants désignés:
**CH DE FR GB IT LI NL**

(56) Documents cités:
**FR-A-2 082 768**
**FR-A-2 293 432**
**FR-A-2 335 511**
**FR-A-2 371 445**

(73) Titulaire: **ROUSSEL-UCLAF**
**35, boulevard des Invalides**
**F-75007 Paris (FR)**

(72) Inventeur: **Martel, Jacques**
**15, rue Douvillez**
**F-93140 Bondy (FR)**
Inventeur: **Tessier, Jean**
**30, rue Jean Moulin**
**F-94300 Vincennes (FR)**
Inventeur: **Teche, André**
**15, rue Godot de Mauroy**
**F-75009 Paris (FR)**
Inventeur: **Girault, Pierre**
**4, rue des Abbesses**
**F-75018 Paris (FR)**

(74) Mandataire: **Tonnellier, Marie-José et al**
**102, route de Noisy Boîte postale no 9**
**F-93230 Romainville (FR)**

**Le dossier contient des informations techniques présentées postérieurement au dépôt de la demande et ne figurant pas dans le présent fascicule.**

**EP 0 060 955 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne de nouveaux uraciles substitués comportant un groupement 2-tétrahydropyranyle, leur procédé de préparation et les compositions pesticides les renfermant.

On avait déjà connaissance de composés uraciles diversement substitués décrits notamment dans les brevets français portant les numéros FR—A—2 082,768, FR—A—2 079 535, FR—A—2 335 511 et possédant des propriétés herbicides.

Les composés de l'invention se distinguent nettement des composés précédents du point de vue de la nature et de la position des substituants ainsi que du point de vue biologique, notamment par leurs propriétés de désherbage sélectif des cultures de coton et de soja.

L'invention a plus précisément pour objet les composés de formule générale (I):

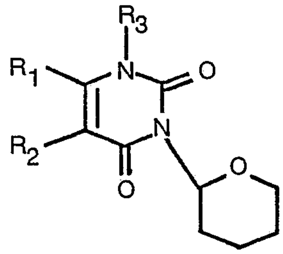

(I)

dans laquelle $R_1$ représente un radical alcoyle, linéaire ou ramifié, comportant de 1 à 3 atomes de carbone, $R_2$ représente un radical alcoyle linéaire ou ramifié comportant de 1 à 3 atomes de carbone, ou bien $R_1$ et $R_2$ représentant ensemble, avec les atomes de carbone auxquels ils sont liés un homocycle carboné comportant de 5 à 7 atomes de carbone et $R_3$ représente *ou bien* un groupement

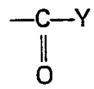

dans laquel Y représente un radical alcoyle, linéaire ou ramifié, comportant de 1 à 6 atomes de carbone, ou un radical phényle éventuellement substitué par un atome d'halogène ou par un radical alcoyle ou alcoxy comportant de 1 à 3 atomes de carbone *ou bien* un groupement —$CO_2Z$ dans lequel Z représente un radical alcoyle linéaire ou ramifié, comportant de 1 à 6 atomes de carbone, un radical cycloalcoyle comportant de 3 à 7 atomes de carbone, un radical phényle ou un radical benzyle, *ou bien* un groupement $SO_2Z_1$, dans lequel $Z_1$ représente un radical alcoyle linéaire ou ramifié, comportant de 1 à atomes de carbone.

Dans les composés de l'invention $R_1$ ou $r_2$ représentent notamment un méthyle, un éthyle ou un propyle ou bien $R_1$ et $R_2$ représentent ensemble avec les atomes de carbone auxquels ils sont liés, soit un cyclopentyle, un cyclohexyle ou un cycloheptyle, soit un cycle benzénique et $R_3$ représente notamment ou bien un groupement

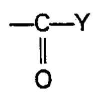

dans lequel Y représente un méthyle, un éthyle, un propyle, un hexyle, un phényle, un p-chlorophényle ou un p-méthoxyphényle, ou bien un groupement —$CO_2Z$ dans lequel Z représente un méthyle, un éthyle, un propyle, un butyle, un hexyle, un cyclopropyle, un cyclopentyle, un cyclohexyle, un radical phényle ou un radical benzyle, ou bien un groupement —$SO_2Z_1$ dans lequel $Z_1$ représente un méthyle, un éthyle, un propyle ou un butyle.

L'invention a plus particulièrement pour object les composés de formule générale (I) dans lesquels $R_1$ et $R_2$ représentent ensemble avec les atomes de carbone auxquels ils sont liés un homocycle carboné comportant de 5 à 7 atomes de carbone et $R_3$ conserve les signification précitées, les composés de formule générale (I), dans lesquels $R_1$ et $R_2$ représentent ensemble avec les atomes de carbone auxquels ils sont liés un homocycle carboné renfermant de 5 à 7 atomes de carbone et $R_3$ représente un groupement $CO_2Z$ dans lequel Z représente un radical alcoyle, linéaire ou ramifié, comportant de 1 à 3 atomes de carbone et notamment le produit de l'exemple 18, c'est-à-dire la (1-méthoxy carbonyl) 3-(2-tétrahydropyranyl) 1,2,3,4,6,7-hexahydro 5H-cyclopentapyrimidine 2,4-dione. L'invention a aussi plus particulièrement pour objet les composés de formule générale (I) dans lesquels $R_1$ et $R_2$ représentent des radicaux alcoyles, linéaires ou ramifiés, comportant de 1 à 3 atomes de carbone et $R_3$ représente un groupement

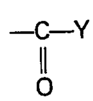

dans lequel Y représente un radical alcoyle, linéaire ou ramifié, comportant de 1 à 6 atomes de carbone ou un radical phényle.

Les composés de l'invention sont doués de remarquables propriétés pesticides notamment herbicides.

Les propriétés herbicides de pré ou de post-émergence des composés de l'invention peuvent être mises en évidence par des tests sur des plantes représentatives de grandes familles botaniques tels que triticum sativum, hordeum species, zea maïs, avena fatua, agrostis tenuis, lolium perenne, alopecurus moysuroïdes, beta vulgaris, chenopodium chinoa, chrysanthemum coronarium, galium aparine, sinapis alba, rumex crispus, trifolium praetense.

L'étude compartive des propriétés herbicides des composés de l'invention sur coton et sur xanthium ainsi que sur soja et sur xanthium permet d'autre part de juger de la sélectivité des composés de l'invention dans le désherbage de ces deux plantes cultivées.

La partie expérimentale exposée ci-après met en évidence l'activité herbicide sélective des composés de l'invention vis-à-vis des graminées cultivées (blé, orge, avoine, maïs).

Les tests montrent également que les composés de l'invention, notamment ceux pour lesquels $R_3$ représente un groupement $—CO_2Z$ dans lequel Z représente un radical alcoyle linéaire ou ramidié, comportant de 1 à 6 et notamment de 1 à 3 atomes de carbone, présentent une activité herbicide sélective vis à vis du coton et du soja, en détruisant les adventices de ces plantes à des doses bien inférieures à celles pour lesquelles se manifeste une phytotoxicité vis à vis des plantes cultivées.

L'invention a également pour objet un procédé de préparation des composés de formule générale (I) tels que définis cidessus, caractérisé en ce que l'on fait réagir sur un composé de formule (II):

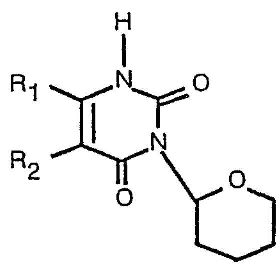

(II)

une base forte puis un composé de formule (III):

$$X—R_3$$

dans laquelle X représente un halogène et de préférence du chlore et $R_3$ conserve les significants précitées.

La base forte en présence de laquelle on condense le composé (III) avec le composé (II) est notamment l'hydrure de sodium ou l'amidure de sodium. La réaction peut être effectuée au sein d'un solvant organique tel que le diméthyl formamide, ou encore le tétrahydrofuranne en présence d'hexaméthylphosphotriamide.

L'invention a plus particulièreemnt pour object un procédé de préparation du 1-(méthoxycarbonyl) 3-(2-tétrahydropyranyl) 1,2,3,4,6,7-hexahydro 5H-cyclopentapyrimidine 2,4-dione, caractérisé en ce que l'on fait réagir sur la 3-(2-tétrahydropyranyl) 1,2,3,4,6,7-hexahydro 5H-cyclopentapyrimidine 2,4-dione, l'hydrure de sodium puis le chloroformiate de méthyle.

Les composés de formule générale (II) utilisés au départ du procédé de l'invention peuvent être préparés en faisant réagir un produit de formule générale:

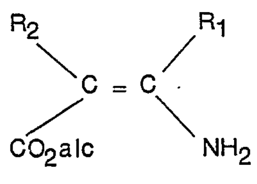

dans laquelle $R_1$ et $R_2$ conservent les significations précitées et alc représente un radical alcoyle inférieur, avec l'isocyanate de 2-tétrahydropyranyle, selon, notamment, les procédés du brevet français FR—A—2 082 768 et du brevent français FR—A—2 293 432.

L'invention a aussi pour objet les compositions pesticides, notamment herbicides, caractérisées en ce qu'elles contiennent, comme principe actif, un au moins des composés de formule générale (I).

L'invention a plus particulièrement pour objet les compositions pesticides, notamment herbicides, caractérisées en ce qu'elles contiennent comme principe actif, un au moins des composés de formule (I) dans lesquels soit $R_1$ et $R_2$ représentent ensemble avec les atomes de carbone auxquels ils sont liés un homocycle carboné comportant de 5 à 7 atomes de carbone et $R_3$ conserve les significations précitées, soit $R_1$ et $R_2$ représentent ensemble avec les atomes de carbone auxquels ils sont liés un homocycle carboné renfermant de 5 à 7 atomes de carbone et $R_3$ représente un groupement $CO_2Z$ dans lequel Z représente un radical alcoyle, linéaire ou ramifié, comportant de 1 à 3 atomes de carbone, soit $R_1$ et $R_2$ représentent des

radicaux alcoyles, linéaires ou ramifiés, comportant de 1 à 3 atomes de carbone et $R_3$ représente un groupment

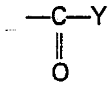

dans lequel Y représente un radical alcoyle, linéaire ou ramifié, comportant de 1 à 6 atomes de carbone ou un radical phényle.

L'invention a plus spécialement pous objet les compositions pesticides, notamment herbicides, caractérisées en ce qu'elles contiennent, comme principe actif la 1-(méthoxycarbonyl) 3-(2-tétrahydropyranyl) 1,2,3,4,6,7-hexahydro 5H-cyclopentapyrimidine-2,4-dione.

Les compositions selon l'invention peuvent se présenter sous forme de poudres, granulés, suspensions, émulsions, solutions contenant le principe actif, par exemple, en mélange avec un véhicule et/ou un agent tensio-actif anionique, cationique ou non ionique assurant entre autres, une dispersion uniforme des substances de la composition. Le véhicule utilisé peut être un liquide, tel que l'eau, l'alcool, les hydrocarbures ou autres solvants organiques, une huile minérale, animale ou végétale, ou une poudre, telle que le talc, les argiles, les silicates, le kieselguhr.

Les compositions solides, présentées sous forme de poudre pour poudrage, de poudres mouillables ou de granulés, peuvent être préparées par broyage du composé actif avec un solide inerte ou par imprégnation d'un support solide avec une solution du principe actif dans un solvant que l'on évapore ensuite.

Outre un véhicule et/ou un agent tensio-actif, ces compositions contiennent, comme principe actif, un ou plusieurs composés de formule (I), ainsi qu'éventuellement d'autres pesticides et des substances présentant des propriétés influant sur la croissance des plantes.

Les compositions de l'invention sont, bien entedu, appliquées à des doses suffisantes pour exercer leurs activités pesticides notamment herbicides. Les doses de matière active dans les compositions varient notamment en fonction des végétaux à détruire, de la nature du terrain, des conditions atmosphériques et de l'état d'avancement de la végétation à détruire.

Les compositions herbicides selon l'invention contiennent en général de 10 à 80% en poids et, de préférence, de 10 à 50% en poids de matière active.

Lors de l'utilisation des composés I comme herbicide, les quantités de substance active appliquées varient, de préférence, entre 0,1 et 5 Kg/ha.

L'invention a également pour objet l'application des composés de formule (I), notamment des composés de formule (I) dans laquelle $R_1$ et $R_2$ représentent ensemble avec les atomes de carbone auxquels ils sont liés un homocycle carboné comportant de 5 à 7 atomes de carbone et $R_3$ représente un groupement $—CO_2Z$ dans lequel Z représente un radical alcoyle, linéaire ou ramifié, comportant de 1 à 3 atomes de carbone et plus particulièrement de la 1-(méthoxycarbonyl) 3-(2-tétrahydropyranyl) 1,2,3,4,6,7-hexahydro 5H-cyclopentapyrimidine 2,4 dione, au désherbage sélectif des cultures de coton, des cultures de soja, ainsi que des cultures de blé, d'orge, de maïs et d'avoine.

Les exemples suivants illustrent l'invention sand la limiter:

## Exemple 1

1-(Carbéthoxy) 3-(2-tétrahydropyranyl 5,6-diméthyl 1,2,3,4-tétrahydropyrimidine 2,4-dione

Dans 1120 cm3 de diméthyl formamide on introduit 224 g de 3-(2'-tétrahydropyranyl) 5,6-diméthyl-1,2,3,4-tétrahydropyrimidine-2,4-dione, puis progressivement à 20°C, 50 g d'une suspension d'hydrure de sodium à 50% dans l'huile de vaseline, agite pendant 2 heures à 20°C, ajoute progressivement 124 g de chloroformiate d'éthyle, agite pendant 20 heures à 20°C, concentre à sec par distillation sous pression réduite, ajoute de l'éther de pétrole au résidu, agite, laisse décanter, élimine l'éther de pétrole, recommence l'opération à plusieurs reprises, concentre à sec sous pression réduite, dissout le résidu dans le chlorure de méthylène, élimine, par filtration, l'insoluble résiduel, chromatographie le filtrat sur silice, cristallise dans l'éther isopropylique et obtient 94,4 g de 1-carbéthoxy 3-(2-tétrahydropyranyl) 5,6-diméthyl 1,2,3,4-tétrahydropyrimidine 2,4-dione.

F = 74°C.

## Exemple 2

1-(1-méthyl éthyl oxycarbonyl) 3-(2-tétrahydropyranyl) 5-éthyl 6-méthyl 1,2,3,4-tétrahydropyrimidine 2,4-dione

Dans 500 cm3 de diméthyl formamide, on introduit 47,6 g de 3-(2-tétrahydropyranyl) 5-éthyl 6-méthyl 1,2,3,4-tétrahydropyrimidine, 2,4-dione, ajoute 10 g de suspension d'hydrure de sodium à 50% dans l'huile de vaseline, agite pendant 15 minutes à 20°C, introduit progressivement, à 20°C, 24,4 g de chloroformiate d'isopropyle, agite pendant 30 minutes à +20°C, verse le mélange réactionnel dans un mélange d'eau et de glace, extaite au chlorure de méthylène, concentre à sec par distillation sous pression réduite, ajoute de l'éther isopropylique au résidu, agite, élimine par filtration l'insoluble formé, concentre le filtrat à sec par distillation sous pression réduite, chromatographie le résidu sur silice en éluant par un mélange de

cyclohexane et d'acétate d'éthyle (1/1), ajoute de l'éther de pétrole (eb. 35—70°C) au résidu cristallisé, essore, sèche et obtient 11 g de 1-(1-méthyléthyloxycarbonyl) 3-(2-tétrahydropyranyl 5-éthyl 6-méthyl 1,2,3,4-tétrahydropyrimidine 2,4-dione.
F = 118°C.

Analyse: $C_{16}$ $H_{24}$ $N_2O_5$ (324,37)

| | C% | H% | N% |
|---|---|---|---|
| Calculé | 59,24 | 7,46 | 8,64 |
| Trouvé | 59,0 | 7,4 | 8,5 |

Exemple 3

1-(éthoxy carbonyl) 3-(2-tétrahydropyranyl) 5-éthyl 6-méthyl 1,2,3,4-tétrahydropyrimidine 2,4-dione

Dans 9 litres de diméthyl formamide on introduit 1,5 kg de 3-(2-tétrahydropyranyl) 5-éthyl 6-méthyl 1,2,3,4-tétrahydropyrimidine 2,4-dione, introduit progressivement à 0°C, 450 g d'une suspension huileuse à 50% d'hydrure de sodium, agite pendant une heure à + 15°C, introduit lentement en une heure trente minutes, à 20°C, 1,35 kg de chloroformiate d'éthyle, agite pendant 20 heures à + 20°C, verse le mélange réactionnel dans un mélange d'eau, de glace et de chlorure de méthylène, agite, décante, extrait à nouveau au chlorure de méthyléne, lave la solution organique successivement avec une solution de soude N, une solution aqueouse saturée de chlorure de sodium puis à l'eau, la sèche, concentre à sec par distillation sous pression réduite, dissout ce résidu dans le benzène, ajoute de la silice colloïdale, agite, filtre pour éliminer la silice, concentre le filtrat à sec par distillation sous pression réduite, dissout le résidu dans l'éther isopropylique, ajoute de l'heptane, agite, isole par essorage, le précipité formé, le cristallise dans l'éther isopropylique et obtient 1,05 kg de 1-(éthoxycarbonyl) 3-(2-tétrahydropyranyl) 5-éthyl 6-méthyl 1,2,3,4-tetrahydropyrimidine 2,4-dione
F = 85°C.

Analyse: $C_{15}$ $H_{22}$ $N_2O_5$ (310,35)

| | C% | H% | N% |
|---|---|---|---|
| Calculé | 58,04 | 7,14 | 9,02 |
| Trouvé | 58,3 | 7,4 | 9,0 |

Spectre UV (éthanol)
max. à 264 nm, ε = 9700.

Exemple 4

1-(méthoxycarbonyl) 3-(2-tétrahydropyranyl) 5-éthyl 6-méthyl 1,2,3,4-tétrahydropyrimidine 2,4-dione

Dans 300 cm3 de diméthylformamide on introduit 48 g de 3-(2-tétrahydropyranyl) 5-éthyl 6-méthyl tétrahydropyrimidine 2,4-dione, ajoute, progressivement 10 g de suspension d'hydrure de sodium à 50% dans l'huile de vaseline, agite pendant une heure et trente minutes à 20°C, ajoute 21 g de chloroformiate de méthyle, agite pendant 16 heures à 20°C, verse le mélange réactionnel dans un mélange d'eau et de glace, extrait au chlorure de méthylène, concentre à sec par distillation sous pression réduite, chromatographie le résidu sur silice en éluant par un mélange de cyclohexane et d'acétate d'éthyle (1—1) et obtient 22 g de 1-(méthoxycarbonyl) 3-(2-tetrahydropyranyl) 5-éthyl 6-méthyl 1,2,3,4-tétrahydropyrimidine 2,4-dione.

Spectre UV (éthanol)
max. à 265nm, ε = 8200
Spectre de RMN (deutérochloroforme)

pics à 0,90—1,02—1,13 ppm atribués aux hydrogènes du méthyle de l'éthyle in position 5.
pic à 2,12 ppm attribué aux hydrogènes du méthyle en position 6.
pics à 2,22—2,33—2,45—2,58 ppm atribués aux hydrogènes du méthylène de l'éthyle en position 5.
pic à 40 ppm attribué aux hydrogènes du méthyle du méthoxy carbonyle.
pics à 5,75—5,78—5,95—5,98 ppm attribués à l'hydrogène en position 1 du cycle 2-tétrahydropyranyle.

Exemple 5

1 (n-hexyloxycarbonyl) 3-(2-tétrahydropyranyl) 5-éthyl 6-méthyl 1,2,3,4-tétrahydropyrimidine 2,4-dione

Dans 500 cm3 de dimethylformamide on introduit 47,6 g de 3-(2-tétrahydropyranyl) 5-éthyl 6-méthyl 1,2,3,4-tétrahydropyrimidine 2,4-dione, ajoute progressivement 10 g de suspension à 50% d'hydrure de sodium dans l'huile, agite pendant une heure à 20°C, introduit lentement 32,9 g de chloroformiate de n-hexyle, agite à 20°C pendant 44 heures, verse le mélange réactionnel dans un mélange d'eau et de glace, extrait au chlorure de méthylène, concentre à sec par distillation sous pression réduite, chromatographie le résidu sur silice en éluant par un mélange de cyclohexane et d'acétate d'éthyle (1/1), puis par un mélange de cyclohexane et d'acétate d'éthyle (8—2) et obtient 12 g de 1 (n-hexyloxycarbonyl) 3-(2-tétra-hydropyranyl) 5-éthyl 6-méthyl 1,2,3,4-tétrahydropyrimidine 2,4-dione.

Spectre de RMN (deutérochloroforme)
pic à 0,9 ppm attribué aux hydrogènes du méthyle de la chaîne hexyle.
pics à 0,93—1,05—1,17 ppm attribués aux hydrogènes du méthyle terminal de l'éthyle en position 5.
pic à 2,14 ppm attribué aux hydrogènes du méthyle en position 6.
pics à 3,33—4,17 ppm attribués aux hydrogènes du méthylène en position 3 du cycle tétrahydropyranique.
pics à 4,28—4,4—4,52 ppm attribués aux hydrogènes du méthylène du groupement hexyle en position α du

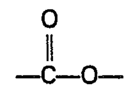

pics à 5,82—5,85—5,98—6,02 ppm attribués à l'hydrogène en position 1 du cycle tétrahydropyranyle.

Exemple 6

1-(propyloxycarbonyl) 3-(2-tétrahydropyranyl) 5-éthyl 6-méthyl 1,2,3,4-tétrahydropyrimidine 2,4-dione

Dans 500 cm3 de diméthylformamide on introduit 47,6 g de 3-(2-tétrahydropyranyl) 5-éthyl 6-méthyl 1,2,3,4-tétrahydropyrimidine 2,4-dione, ajoute lentement 10 g de suspension à 50% d'hydrure de sodium dans l'huile, agite pendant 15 minutes à +20°C ajoute progressivement à + 20°C, 25 g de chloroformiate de propyle, agite pendant 30 minutes à 20°C, verse le mélange réactionnel dans un mé;ange d'eau et de glace, élimine par filtration l'insoluble formé, concentre le filtrat à sec par distillation sous pression réduite, chromatographie le résidu sur silice en éluant avec un mélange de cyclohexane et d'acétate d'éthyle (1—1) et obtient 20 g de 1-(propyloxycarbonyl) 3-(2-tétrahydropropanyl) 5-éthyl 6-méthyl 1,2,3,4-tétrahydropyrimidine 2,4-dione.

Analyse: $C_{16}H_{24}N_2O_5$ (324,37)

| | C% | H% | N% |
|---|---|---|---|
| Calculé | 59,24 | 7,46 | 8,64 |
| Trouvé | 59,2 | 7,7 | 8,2 |

Exemple 7

1-(éthoxycarbonyl)3-(2-tétrahydropyranyl) 5-isopropyl 6-méthyl 1,2,3,4-tétrahydropyrimidine 2,4-dione.

Dans 200 cm3 de diméthylformamide on introduit 35,8 g de 3-(2-tétrahydropyranyl) 5-isopropyl 6-méthyl 1,2,3,4-tétrahydropyrimidine 2,4-dione, ajoute lentement 6,8 g d'une suspension à 50% d'hydrure de sodium dans l'huile, agite pendant 45 minutes à + 20°C, introduit progressivement 30,8 g de chloroformiate d'éthyle, agite pendant 72 heures à 20°C, verse le mélange réactionnel dans l'eau, extrait au chloroforme, concentre à sec par distillation sous pression réduite, chromatographie le résidu sur gel de silice en éluant par un mélange de benzène et d'acétate d'éthyle (4—6) et obtient 28,5 g de 1-(éthoxycarbonyl) 3-(2-tétrahydropyranyl) 5-isopropyl 6-méthyl 1,2,3,4-tétrahydropyrimidine 2,4-dione.

Spectre de RMN (deutérochloroforme).
pics à 1,21—1,32 ppm attribués aux hydrogènes des méthyles de l'isopropyle.
pics à 1,30—1,42—1,53 ppm attribués aux hydrogènes du méthyle de $—CO_2C_2H_5$
pics à 4,28—4,40—4,52—4,63 ppm attribués aux hydrogènes du méthylène de $—CO_2C_2H_5$
pic à 2,14 ppm attribué aux hydrogènes de

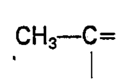

Exemple 8

1-(cyclohexyloxycarbonyl) 3-(2-tétrahydropyranyl) 5-éthyl 6-méthyl 1,2,3,4-tétrahydropyrimidine 2,4-dione.

Dans 500 cm3 de diméthylformamide on introduit 47,6 g de 3-(2-tétrahydropyranyl) 5-éthyl 6-méthyl 1,2,3,4-tétrahydropyrimidine 2,4-dione, ajoute lentement 10 g d'une suspension à 50% d'hydrure de sodium dans l'huile, ajoute goutte à goutte à + 20°C, 32,4 g de chloroformiate de cyclohexyle, agite pendant 30 minutes à + 20°C, verse le mélange réactionnel dans un mélange d'eau et de glace, extrait au chlorure de méthylène, concentre à sec par distillation sous pression réduite, cristallise le résidu dans l'acétate d'éthyle, élimine par filtration l'insoluble formé, concentre le filtrat à sec par distillation sous pression réduite, chromatographie le résidu sur silice en éluant par un mélange de cyclohexane et d'acétate d'éthyle (1—1) et obtient 26 g de 1-(cyclohexyloxycarbonyl) 3-(2-tétrahydropyranyl) 5-éthyl 6-méthyl 1,2,3,4-tétrahydropyrimidine 2,4-dione.

Spectre DE RMN (deutérochloroforme)
pics à 0,92—1,05—1,17 ppm attribués aux hydrogènes du méthyle, de l'éthyle en position 5.
pics à 2,25—2,37—2,48—2,60 ppm attribués aux hydrogènes du méthylène de l'éthyle en position 5.
pic à 2,15 ppm attribué aux hydrogènes de

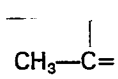

pics à 3,17—4,33 ppm attribués au méthylène en position 3 du cycle tétrahydropyranyle.
pics de 3,0—4,35 ppm attribués aux autres protons du cycle tétrahydropyranyle.
pic à 4,9 ppm attribué à l'hydrogène en position du cyclohexane.
pics à 5,78—5,81—5,97—6,0 ppm attribués à l'hydrogène en position 1 du tétrahydropyranyle.

### Exemple 9

1-(phénoxycarbonyl) 3-(2-tétrahydropyranyl) 5-éthyl 6-méthyl 1,2,3,4-tétrahydropyrimidine 2,4-dione.

Dans 250 cm3 de diméthylformamide on introduit 23,8 g de 3-(2-tétrahydropyranyl) 5-éthyl 6-méthyl 1,2,3,4-tétrahydropyrmidine 2,4-dione, ajoute lentement 5 g d'une suspension d'hydrure de sodium à 50% dans l'huile, agite pendant 30 minutes, ajoute 15,6 g de chloroformiate de phényle, agite pendant 30 minutes à 20°C, verse le mélange réactionnel dans l'eau, extrait au chlorure de méthylène, concentre à sec par distillation sous pression réduite, chromatographie le résidu sur silice en éluant avec un mélange de cyclohexane et d'acétate d'éthyle (7—3) et obtient 11 g de 1-(phénoxycarbonyl) 3-(2-tétrahydropyranyl) 5-éthyl 6-méthyl 1,2,3,4-tétrahydropyrimidine 2,4-dione.

Analyse: $C_{19} H_{22} N_2O_5$ (358,42)

| | C% | H% | N% |
|---|---|---|---|
| Calculé | 63,66 | 6,19 | 7,82 |
| Trouvé | 63,6 | 6,5 | 7,6 |

Spectre RMN (deutérochloroforme)
pics à 0,95—1,07—1,18 ppm attribués aux hydrogènes du méthyle de l'éthyl en position 5.
pic à 2,27 ppm attribués aux hydrogènes du méthyle en position 6.
pics à 2,28—2,40—2,52—2,63 ppm attribués aux hydrogènes du méthylène de l'éthyl en position 5.
pics de 3,17 à 4,33 ppm attribués aux hydrogènes du méthylène en position 3 du cycle tétrahydropyranyle.
pics à 5,83—5,93—6,02—6,05 ppm attribués à l'hydrogène en position 1 du cycle tétrahydropyranyle.
pic à 7,36 ppm attribués aux hydrogènes du noyau aromatique.

### Exemple 10

1-(benzyloxycarbonyl) 3-(2-tétrahydropyranyl) 5-éthyl 6-méthyl 1,2,3,4-tétrahydropyrimidine 2,4-dione.

Dans 300 cm3 de diméthylformamide, on introduit 35,7 g de 3-(2-tétrahydropyranyl) 5-éthyl 6-méthyl 1,2,3,4-tétrahydropyrimidine 2,4-dione, ajoute progressivement 7,2 g de suspension d'hydrure de sodium à 50% dans l'huile, agite pendant une heure à 25°C, ajoute goutte à goutte 50 g de chloroformiate de benzyle, agite pendant 4 heures à 25°C, verse le mélange réactionnel dans l'eau, extraite au chlorure de méthylène, concentre à sec par distillation sous pression réduite, chromatographie le résidu sur silice en éluant par un mélange de cyclohexane et d'acétate d'éthyle (1—1) et obtient 10 g de 1-(benzyloxycarbonyl) 3-(2-tétrahydropyranyl) 5-éthyl 6-methyl 1,2,3,4-tétrahydropyrimidine 2,4-dione.

Spectre de RMN (deuterochloroforme)
pics à 0,9—1,03—1,15 ppm attribués aux hydrogènes du méthyle de l'éthyle en position 5.
pics à 1,33—2,0 ppm attribués aux hydrogènes des méthylènes en position 4,5,6 du cycle tétrahydropyranyle.
pic à 2,02 ppm attribués aux hydrogènes du méthyle en position 6.
pics à 3,0—4,17 ppm attribués aux hydrogènes du méthylène en position 3 du cycle tétrahydropyranyle.
pic à 5,47 ppm attribués aux hydrogènes du méthylène du benzyle.
pics à 5,87—6,05 ppm attribués à l'hydrogène en position 1 du cycle tétrahydropyranyle.
pics à 7,3387,5 ppm attribués aux hydrogènes des noyaux aromatiques.

### Exemple 11

1-(carbéthoxy) 3-(2-tétrahydropyranyl) 1,2,3,4,6,7-hexahydro 5H cyclopentapyrimidine 2,4-dione.

Dans 900 cm3 de diméthylformamide on dissout 138 g de 3-(2-tétrahydropyranyl) 1,2,3,4,6,7-hexahydro 5H cyclopentapyrimidine 2,4-dione, ajoute lentement 44 g d'une suspension d'hydrure de sodium à 50% dans l'huile, agite pendant 2 heures à + 20°C, ajoute progressivement à + 20°C, 135,6 g de chloroformiate d'éthyle, agite pendant 40 heures à + 20°C, verse le mélange, réactionnel dans un mélange d'eau et de glace, extrait au chlorure de méthylène, concentre à sec par distillation sous pression réduite, ajoute au résidu de l'éther de pétrole (eb 35—75°C), agite, élimine l'éther de pétrole, recommence la même opération, dissout le résidu dans l'éther éthylique, chromatographie sur silice, en éluant à l'éther éthylique de obtient 158 g de 1-(carbéthoxy) 3-(2-tétrahydropyranyl) 1,2,3,4,6,7-hexahydro 5H-cyclopentapyrimidine 2,4-dione.

Spectra UV (éthanol)
max. 267 nm, ε 9000.

Exemple 12

1-(acétyl) 3-(2-tétrahydropyranyl) 5-éthyl 6-méthyl 1,2,3,4-tétrahydropyrimidine 2,4-dione

Dans 300 cm3 de diméthyl formamide on introduit 54,7 g de 3-(2-tétrahydropyranyl) 5-éthyl 6-méthyl 1,2,3,4-tétrahydropyrimidine 2,4-dione, ajoute, lentement, 11,4 g de suspension d'hydrure de sodium à 50% dans l'huile, agite pendant 2 heures à 20°C, ajoute lentement 18,7 g de chlorure d'acétyle, agite pendant 80 heures, verse le mélange réactionnel sur un mélange d'eau et de glace, extrait à l'acétate d'éthyle, concentre à sec par distillation sous pression réduite, ajoute au résidu 400 cm 3 d'un mélange de cyclohexane et d'acétate d'éthyle (1—1), élimine par filtration l'insoluble formé, chromatographie le filtrat sur silice en éluant par un mélange de cyclohexane et d'acétate d'éthyle (1—1), concentre à sec par distillation sur pression réduite, lave le concentrat à l'éther de pétrole (eb 35—75°C) et obtient 13,9 g de 1-(acétyle) 3-(2-tétrahydropyranyl) 5-éthyl 6-méthyl 1,2,3,4-tétrahydropyrimidine 2,4-dione, F = 80°C.

Après cristallisation dans l'acétate d'éthyle on obtient un produit (F = 80°C), dont les caractéristiques sont les suivantes:

Analyse: $C_{14}$ $H_{20}$ $N_2O_5$ (280,32)

| | C% | H% | N% |
|---|---|---|---|
| Calculé | 59,99 | 7,19 | 9,99 |
| Trouvé | 60,0 | 7,2 | 10,0 |

Spectre de RMN (deutérochloroforme)

pics à 0,93—1,07—1,2 ppm et 2,28—2,4—2,53—2,67 ppm attribués aux hydrogènes

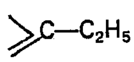

pic à 2,01 ppm attribué à $—COCH_3$.

pic à 2,67 ppm attribué à

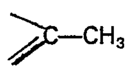

pics à 2,66—3,33 ppm attribués à $—CH_2O—$ du tétrahydropyranyle.

pics à 5,87—6,05 ppm attribués à l'hydrogène angulaire du tétrahydropyranyle.

Exemple 13

1-(benzoyl) 3-(2-tétrahydropyranyl) 5-éthyl 6-méthyl 1,2,3,4-tétrahydropyrimidine 2,4-dione.

Dans 100 cm3 de diméthyl formamide on introduit 23,8 g de 3-(2-tétrahydropyranyl) 5-éthyl 6-méthyl tétrahydropyrimidine 2,4-dione, ajoute progressivement 4,6 g de suspension d'hydrure de sodium à 50% dans l'huile, agite pendant 1 heure, ajoute goutte à goutte 15,4 g de chlorure de benzoyle, agite pendant 66 heures à 20°C, verse le mélange réactionnel sur un mélange d'eau et de glace, extrait à l'acétate d'éthyle, concentre à sec sous pression réduite, chromatographie le résidu sur silice en éluant par un mélange de cyclohexane et d'acétate d'éthyle (1—1) et obtient 19,6 g de 1-benzoyl 3-(2-tétrahydropyranyl) 5-éthyl 6-méthyl 1,2,3,4-tétrahydropyrimidine 2,4-dione, F = 154°C.

Après cristallisation dans l'acétate d'éthyle le produit obtenu (f = 154°C) présente les caractéristiques suivantes:

Analyse: $C_{19}$ $H_{22}$ $N_2O_4$ (342,39)

| | C% | H% | N% |
|---|---|---|---|
| Calculé | 66,64 | 6,48 | 8,18 |
| Trouvé | 66,4 | 6,6 | 8,1 |

Spectre de RMN (deutérochloroforme)

pics à 1,0—1,12—1,23 ppm attribués aux hydrogènes du méthyle de l'éthyl en position 5.

pic à 1,92 ppm attribués aux hydrogènes du méthyle en position 6.

pics à 2,33—2,45—2,53—2,58 ppm attribués aux hydrogènes du méthylène de l'e´thyl en position 5.

pics à 3,33—4,33 ppm attribués à $—CH_2—O—$.

pics à 6,05—6,38 ppm attribués à l'hydrogène angulaire du tétrahydropyrane.

pics à 7,95—8.13 ppm attribués aux hydrogènes du phényle en a du C=O.

pics à 7,42—7,83 ppm attribués aux autres hydrogènes aromatiques.

Exemple 14

1-(méthyl sulfonyl) 3-(2-tétrahydropyranyl) 1,2,3,4,6,7-hexahydro 5H-cyclopentapyrimidine 2,4-dione

Dans 300 cm3, de diméthyle formamide on introduit 46 g de 3-(2-tétrahydropyranyl) 1,2,3,4,6,7-hexahydro 5H cyclopentapyrimidine 2,4-dione, ajoute lentement 10 g de suspension à 50% d'hydrure de sodium dans l'huile, agite pendant 30 minutes à +20°C, ajoute progressivement 25 g de chlorure de méthane sulfonyle, agite à +20°C pendant 16 heures, verse le mélange réactionnel dans l'eau extrait à l'acétate d'éthyle, concentre à sec par distillation sous pression réduite, ajoute de l'éther isopropylique au résidu, agite, isole par essorage le précipité formé, le cristallise dans l'éthanol et obtient 24 g de 1-(méthyl sulfonyl) 3-(2-tétrahydropyranyl) 1,2,3,4,6,7-hexahydro 5H-cyclopentapyrimidine 2,4-dione, F =156°C.

Analyse: $C_{13}$ $H_{18}$ $N_2O_5S$ (314, 36)

| | C% | H% | N% | S% | O% |
|---|---|---|---|---|---|
| Calculé | 49,67 | 5,77 | 8,91 | 10,20 | 25,45 |
| Trouvé | 49,4 | 5,8 | 8,6 | 10,3 | 25,6 |

Exemple 15

1-méthoxycarbonyl 3-(2-tétrahydropyranyl) 5,6-diméthyl 1,2,3,4-tétrahydropyrimidine 2,4-dione

On opère comme indiqué à l'exemple 1, au départ de 25 g de 3-(2-tétrahydropyranyl) 5,6-diméthyl 1,2,3,4-tétrahydropyrimidine 2,4-dione et de 11,5 g de chloroformiate de méthyle et obtient après chromatographie sur silice (éluant: Hexaneacétate d'éthyle 7/3), 3,4 g de produit attendu. F = 110°C.

Analyse: $C_{13}$ $H_{18}$ $N_2O_5$ (282,29)

| | C% | H% | N% |
|---|---|---|---|
| Calculé | 55,31 | 6,43 | 9.93 |
| Trouvé | 55,2 | 6,5 | 9,8 |

Exemple 16

1-phénoxycarbonyl 3-(2-tétrahydropyranyl) 5,6-diméthyl 1,2,3,4-tétrahydropyrimidine 2,4-dione

On opère comme indiqué à l'exemple 9, au départe de 22,5 g de 3-(2-tétrahydropyranyl) 5,6-diméthyl 1,2,3,4-tétrahydropyrimidine 2,4-dione et de 15,6 g de chloroformiate de phényle et obtient après chromatographie sur silice (éluant: cyclohexaneacétate d'éthyl 7/3) 7,7 g de produit attendu. F = 122°C.

Analyse: $C_{18}$ $H_{20}$ $N_2O_5$ (344,36)

| | C% | H% | N% |
|---|---|---|---|
| Calculé | 62,78 | 5,85 | 8.14 |
| Trouvé | 62,6 | 5,9 | 7,9 |

Exemple 17

1-phénoxycarbonyl 3-(2-tétrahydropyranyl) 1,2,3,4,6,7-hexahydro 5H-cyclopentapyrimidine 2,4-dione

On opère de mainère analogue à celle décrite à l'exemple 11, au départ de 35 g de 3-(2-tétrahydropyranyl) 1,2,3,4,6,7-hexahydro 5H-cyclopentapyrimidine 2,4-dione et de 25 g de chloroformiate de phényle et obtient après chromatographie sur silice (éluant: cyclohexane-acétate d'éthyle 7/3) 3,37 g de produit attendu. F = 126°C.

Analyse: $C_{19}$ $H_{20}$ $N_2O_5$ (356,37)

| | C% | H% | N% |
|---|---|---|---|
| Calculé | 64,03 | 5,66 | 7,86 |
| Trouvé | 63,4 | 5,7 | 7,9 |

Exemple 18

1-méthoxycarbonyl 3-(2-tétrahydropyranyl) 1,2,3,4,6,7-hexahydro 5H-cyclopentapyrimidine 2,4-dione

On opère de manière analogue à celle décrite à l'exemple 11, au départ de 15 g de 3-(2-tétrahydropyranyl) 1,2,3,4,6,7-hexahydro 5H-cyclopentapyrimidine 2,4-dione et de 5,9 cm 3 de chloroformiate de méthyle, en opérant dans le tétrahydrofuranne en présence d'hexaméthylphosphotriamide au lieu du diméthylformamide. On Obtient après chromatographie sur silice (éluant: cyclohexane-acétate d'éthyle 5/5) 4,9 g de produit attendu.

Analyse: $C_{14}H_{18}N_2O_5$ (294, 30)

| | C% | H% | N% |
|---|---|---|---|
| Calculé | 57,13 | 6,16 | 9,52 |
| Trouvé | 57,1 | 6,3 | 9,3 |

Spectre RMN: $CDCl_3$ ppm
4,0: H de $CO_2CH_3$.
5,9: H angulaire du radical tétrahydropyranyle.

3,5 à 3,8 }
4 à 3,3 } H de $CH_2O$

2,64 à 3,0 : H de

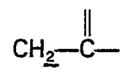

1,6 à 2,9: les autres $CH_2$ du radical tétrahydropyranyle.

Exemple 19

1-acétyl 3-(2-tétrahydropyranyl) 1,2,3,4,6,7-hexahydro 5H-cyclopentapyrimidine 2,4-dione

On opère de manière analogue à celle décrite à l'exemple 11, au départ de 47 g de 3-(2-tétrahydropyranyl) 1,2,3,4,6,7-hexahydro 5H-cyclopentapyrimidine 2,4-dione et de 20 g de chlorure d'acétyle et obtient après chromatographie sur silice (éluant: cyclohexane-acétate d'éthyle 7/3) 28 g de produit attendu.

F = 86°C.

Analyse: $C_{14}H_{18}N_2O_5$ (278,31)

| | C% | H% | N% |
|---|---|---|---|
| Calculé | 60,42 | 6,52 | 10,07 |
| Trouvé | 60,2 | 6,5 | 10,1 |

Spectre IR ($CHCl_3$)
Absorptions à 1709. 1667 $cm^{-1}$ (C = O). Présence de C—OC cyclique.
Absence de NH.

Exemple 20

1-phénoxycarbonyl 3-(2-tétrahydropyranyl) 5,6-diéthyl 1,2,3,4-tétrahydropyrimidine 2,4-dione

On opére de manière analogue à celle décrite à l'exemple 9, au départ de 35 g de 3-(2-tétrahydropyranyl) 5,6-diéthyl 1,2,3,4-tétrahydropyrimidine 2,4-dione (décrite dans le brevet français 2 321 494) et de 21,9 g de chloroformiate de phényle et obtient aprés chromatographie sur silice (éluant: cyclohexane-acétate d'éthyle 8/2) 25,4 g de produit attendu.
F = 80°C.

Analyse: $C_{20}H_{24}N_2O_5$ (372,41)

| | C% | H% | N% |
|---|---|---|---|
| Calculé | 64,50 | 6,50 | 7,52 |
| Trouvé | 64,2 | 6,5 | 7,2 |

Exemple 21

1-valéryl 3-(2-tétrahydropyranyl) 5-éthyl 6-méthyl 1,2,3,4-tétrahydropyrimidine 2,4-dione

On opère de manière analogue à celle décrite à l'exemple 6, au départ de 20 g de 3-(2-tétrahydropyranyl) 5-éthyl 6-méthyl 1,2,3,4-tétrahydropyrimidine 2,4-dione et de 11,2 g de chlorure de n-valéryle. On obtient après chromatographie sur silice (éluant: chlorure de méthylène-acétate d'éthyle 9/1 puis cyclohexane-acétate d'éthyle 7/3) 4,1 g de produit attendu.

$n_D^{21}$ =1,5146.

## Exemple 22

1-p-chlorobenzoyl 3-(2-tétrahydropyranyl) 5-éthyl 6-méthyl 1,2,3,4-tétrahydropyrimidine 2,4-dione

On opère de manière analogue à celle décrite à l'exemple 6, au départ de 20 g de 3-(tétrahydropyranyl) 5-éthyl 6-méthyl 1,2,3,4-tétrahydropyrimidine 2,4-dione et de 16,3 g de chlorure de p-chlorobenzoyle et obtient après cristallisation puis re-cristallisation dans l'isopropanol 12,35 g de produit attendu.
F = 176°C.

Analyse: $C_{19}\ H_{21}\ Cl\ N_2O_4$ (376,837)

| | C% | H% | Cl% | N% |
|---|---|---|---|---|
| Calculé | 60,55 | 5,62 | 9,41 | 7,44 |
| Trouvé | 60,2 | 5,5 | 9,8 | 7,2 |

## Exemple 23

1-p-méthoxybenzoyl 3-(2-tétrahydropyranyl) 5-éthyl 6-méthyl 1,2,3,4-tétrahydropyrimidine 2,4-dione

On opére de manière analogue à celle décrite à l'exemple 6, au départ de 3-(2-tétrahydropyranyl) 5-éthyl 6-méthyl 1,2,3,4-téhydropyrimidine 2,4-dione et de chlorure de p-méthoxybenzoyle et obtient le produit attendu.
F = 152°C.

Analyse: $C_{20}\ H_{24}\ N_2O_5$

| | C% | H% | N% |
|---|---|---|---|
| Calculé | 64,50 | 6,50 | 7,52 |
| Trouvé | 64,2 | 6,6 | 7,4 |

Spectre IR (Chloroforme)
Absorption à 1726—1701 $cm^{-1}$ (C = O), à 1693—1635 $cm^{-1}$ (C = C conjuguée), à 1598—1573—1507 $cm^{-1}$ (aromatiques).

## Exemple 24

1-butoxycarbonyl 3-(2-tétrahydropyranyl) 5-éthyl 6-méthyl 1,2,3,4-tétrahydropyrimidine 2,4-dione

On opère de manière analogue à celle décrite à l'example 6, au départ de 3-(2-tétrahydropyranyl) 5-éthyl 6-méthyl 1,2,3,4-tétrahydropyrimidine 2,4-dione et de chloroformiate de butyle et obtient le produit attendu.

Analyse: $C_{17}\ H_{26}\ N_2O_5$

| | C% | H% | N% |
|---|---|---|---|
| Calculé | 60,33 | 7,74 | 8,28 |
| Trouvé | 60,6 | 7,8 | 8,2 |

Spectre RMN ($CDCl_3$)
Pics de 1,26 à 1,7 ppm attribués aux H du méthyle de l'éthyle en 5;
Pic à 3,2 ppm attribué aux H du méthyle en 6;
Pics à 3,45, 3,56, 3,7 et 3,81 ppm attribués aux h du $CH_2$ de l'éthyle en 5;
Pics de 5,16 à 5,81 ppm attribués aux H du $CH_2$ en α de l'oxygène;
Pics à 8,73, 8,76, 8,93 et 8,96 ppm attribués à l'hydrogène an gulaire;
Pics de 1,92 à 3 ppm attribués aux H des autres méthylènes.

## Exemple 25

1,méthoxycarbonyl 3-(2-tétrahydropyranyl) 1,2,3,4-tétrahydroquinazoline 2,4-dione

On opère de manière analogue à celle décrite à l'examplе 18, départ de 15 g de 3-(2-tétrahydropyranyl) 1,2,3,4-tétrahydroquinazoline 2,4-dione et de 5,7 cm 3 de chloroformiate de méthyle et obtient après chromatographie sur silice (éluant: cyclohexane-acétate d'éthyle 5/5 puis 7/3) 8,5 g de produit attendu.

Analyse: $C_{15}\ H_{16}\ N_2O_5$ (304,305)

| | C% | H% | N% |
|---|---|---|---|
| Calculé | 59,21 | 5,30 | 9,20 |
| Trouvé | 59,2 | 5,4 | 8,9 |

La 3-(2-tétrahydropyranyl) 1,2,3,4-tétrahydroquinazoline 2,4-dione utilisée au départ de l'example 25 a été préparée de la manière suivante:

Stade A: N-(2-méthoxycarbonyl) phényl N'-(2-tétrahydropyranyl)urée

On mélange 21,8 g d'anthranilate de méthyle, 500 cm 3 de tétrahydrofuranne, 35 cm 3 de triéthylamine et 32 g d'isocyanate de tétrahydropyranyle. On porte au reflux pendant 16 heures, évapore le tétrahydrofuranne sous pression réduite, refroidit cristallise dans l'éther isopropylique et obtient 33,5 g de produit attendu brut. F = 137°C.

Stade B: 3-(2-tétrahydropyranyl) 1,2,3,4-tétrahydroquinazoline 2,4-dione

On mélange 27 g du produit obtenue au stade A, 380 cm3 de méthanol et 15 g de méthylate de sodium, porte au reflux pendant 1 heure, chasse le méthanol sous pression réduite, reprend le résidu à l'eau, lave à l'éther, acidifie par addition d'acide acétique et essore les cristaux formés. On recristallise le produit dans l'éthanol et obtient 12 g de produit attendu.
F = 240°C.

Analyse: $C_{13}H_{15}N_2O_3$ (246,27)

| | C% | H% | N% |
|---|---|---|---|
| Calculé | 63,39 | 5,73 | 11,38 |
| Trouvé | 62,8 | 5,7 | 11,5 |

Exemple 26

Etrude de l'activité herbicide des composés de l'invention.

1) Tests utilisés.

Les principaux tests utilisés dans l'étude herbicide des composés de l'invention sont décrits ci-après.

A) Etude de l'activité herbicide de pré-cu de post-émergence.

Les végétaux utilisés (Avena Sativa, Avena Fatua, Triticum Sativum, Hordeum Spec., Zea Maïs, Agrostis Tenuis, Lolium Perenne, Alopecurus Myosuroïdes, Beta Vulgaris, Chenopodium Chinoa, Chrysanthemum Coronarium, Sinapis Alba, Rumex Crispus, Trifolium Praetense, Galium Aparine) sont clutivés en bac de culture (23 × 14 × 3 cm), à double fond, l'arerosage s'effectuant par dessous. Les espèces sont placées, à raison de 20 graines par espèce, en lignes espacées de 3 cm, dans un bac unique, et il y a 4 essais pour chaque concentration. Les conditions de culture sont les suivantes: température: 20°C ± 2°C, humidité: 60% environ, éclairement: par tube fluorescent (lumière du jour + blanc brillant) de six heures à vingt-deux heures, chaque jour. Le mélange terreux utilisé est composé de 10 volumes de terre franche, de 10 volumes de sable de rivière et de 2-volumes de tourbe.

Pour les essais de pré-émergence, le traitement est effectué vingt-quatre heures après le semis et le premier arrosage est effectué par aspersion de façon à entraîner une partie du produit au niveau des graines.

Le traitement de post-émergence est effectue sur les parties aériennes, après vingt et un jours de culture.

Le produit à étudier est appliqué, dans des conditions standard, à l'aide d'un micro-pulvérisateur, à des doses correspondant à 5, 2,5—1,25, 0,625 et 0,312 Kg/ha et une dilution correspondant à 560 l/ha.

On effectue un essai avec un témoin non traité. Cet essai comporte le même nombre de plantules que les plantules traitées.

Le contrôle final est effectué par dénombrement de plantules vingt et un jours après traitement.

Les résultats sont exprimés en pourcentage de réduction du nombre de plants (pourcentage de mortalité) M:

$$M = \frac{\text{Nombre de plantules témoin—nombre de plantules traitées toujours vivantes}}{\text{Nombre de plantules témoin}} \times 100$$

On peut également exprimer les résultats en pourcentage de réduction de la végétation (R.P.).

Le type d'étude permet de mettre en évidence la possibilité de désherbage sélectif des graminés cultivées (avoine, blé, maïs, orge).

B) Etude de l'activité herbicide comparée sur coton et sur xanthium, ou sur soja et sur xanthium.

Dans une salle tropicalisée on fait pousser en pot des plantes de coton (ou de soja) et des plants de xanthium.

Dans les essais de pré-émergence on effectue le traitement à la surface du sol à l'aide d'un pulvérisateur.

Dans les essais de post-émergence on effectue le traitement par pulvérisation sur le plant au stade "deux feuilles" et pour le xanthium au bout de 3 semaines (plants de 10 cm environ).

Les numérations sont effectuées au bout de 45 jours après le traitement.

On exprime les résultats en pourcentage de mortalité (M) ou en pourcentage de réduction de poids (R.P.).

2) Résultats obtenus pour divers composés de l'invention

A) *Le 1-(méthoxycarbonyl) 3-(2-tétrahydropyranyl) 5-éthyl 6-méthyl 1,2,3,4-tétrahydropyrimidine 2-4, dione.*

a) Tests de pré-émergence

| Plantes traitées | DOSES 5 kg/ha | 2,5 kg/ha | 1,25 kg/ha | 0,625 kg/ha |
|---|---|---|---|---|
| Triticum Sativum | M: 0<br>R.P. 56 | M: 0<br>R.P. 52 | M: 0<br>R.P. 53 | M: 0<br>R.P. 0 |
| Hordeum Spec. | M: 0<br>R.P. 68 | M: 0<br>R.P. 65 | M: 0<br>R.P. 40 | M: 0<br>R.P. 36 |
| Zea Maïs | M: 0<br>R.P. 30 | M: 0<br>R.P. 63 | M: 0<br>R.P. 37 | M: 0<br>R.P. 23 |
| Avena Fatua | M: 0<br>R.P. 54 | M: 0<br>R.P. 57 | M: 0<br>R.P. 15 | M: 0<br>R.P. 21 |
| Agrostis Tenuis | M: 100 | M: 100 | M: 100 | M: 100 |
| Lolium Perenne | M: 100 | M: 100 | M: 100 | M: 100 |
| Alopecurus Myosuroïdes | M: 100 | M: 62<br>R.P. 89 | M: 100 | M: 0<br>R.P. 80 |
| Beta Vulgaris | M: 100 | M: 100 | M: 100 | M: 100 |
| Chrysanthemum Coronarium | M: 100 | M: 100 | M: 100 | M: 100 |
| Galium Aparine | M: 100 | M: 60<br>R.P. 72 | M: 57<br>R.P. 71 | M: 0 |
| Sinapis Alba | M: 100 | M: 100 | M: 100 | M: 100 |
| Rumex Crispus | M: 100 | M: 100 | M: 100 | M: 100 |
| Trifolium Praetense | M: 100 | M: 100 | M: 100 | M: 100 |

En pré-émergence, une bonne activité sélective pour désherber les graminés cultivées a été trouvée.

b — Test d'activité herbicide comparée sur coton, soja et xanthium
Test de post-émergence.

| | COTON | | | | SOJA | | | | XANTHIUM | | | |
|---|---|---|---|---|---|---|---|---|---|---|---|---|
| Doses en Kg MA/Ha | 1,0 | 0,635 | 0,312 | 0,156 | 1,0 | 0,635 | 0,312 | 0,156 | 1,0 | 0,635 | 0,312 | 0,156 |
| Critères | | M RP | M RP | M P | M RP | M RP | M RP | M RP | | M | M | M |
| | | 0 18,5 | 0 0 | 0 0 | 100 | 25 30 | 0 0 | 0 0 | | 100 | 100 | 100 |

Test de pré-émergence.

| | COTON | | | | SOJA | | | | XANTHIUM | | | |
|---|---|---|---|---|---|---|---|---|---|---|---|---|
| Doses en Kg MA/Ha | 1,25 | 1,0 | 0,5 | 0,25 | 1,25 | 0,625 | 0,312 | 0,156 | 1,25 | 1,0 | 0,5 | 0,25 |
| Critères | M RP | M RP | M | M | M | M RP | M | M | | M | M | M |
| | | 0 12 | 0 | 0 | 100 | 50 35 | 0 | 0 | | 100 | 100 | 100 |

*Conclusion:* En post-émergence comme en pré-émergence le coton et le soja sont peu attaqués ou inattaqués, tandis que le xanthium est détruit.

B) 1-(benzyloxycarbonyl) 3-(2-tétrahydropyranyl) 5-éthyl 6-méthyl 1,2,3,4-tétrahydropyrimidine 2,4-dione

En pré-émergence, on a trouvé une bonne activité sélective pour désherber les graminés cultivées. En post-émergence, on a trouvé une activité herbicide élévée tant vis à vis des graminés que des adventices dicotyladenes. Le maïs, le blé et l'orge présentent toutefois une résistance suffisante pour envisager un désherbage sélectif. Il est également possible d'employér le produit dans la culture du coton et du soja; en effet le produit est particulièrement actif vis à vis d'une adv entice très gênante de ces cultures: l'Hordeum Species.

C) 1-(carbethoxy) 3-(tétrahydropyranyl) 1,2,3,4,6,7-hexahydro 5H-cyclopentapyrimidine 2,4-dione
Test de pré-émergence:

Le Composé testé est doué d'une bonne activité herbicide sélective permettant notamment de désherber le blé et le maïs.

D) 1-(n-hexyloxycarbonyl) 3-(2-tétrahydropyranyl) 5-éthyl 6-méthyl 1,2,3,4-tétrahydropyrimidine 2,4-dione
Test d'activité herbicide comparée sur coton et xanthium (pré-émergence).

Le produit présente une bonne activité sélective, le xanthium étant détruit et le coton peu ou pas attaqué.

E) 1-(éthoxycarbonyl) 3-(2-tétrahydropyranyl) 5-éthyl 6-méthyl 1,2,3,4-tétrahydropyrimidine 2,4-dione
a) Test de pré-émergence

En pré-émergence, on note une sélectivité appréciable vis-à-vis des graminées cultivées et notamment vis-à-vis du maïs.

b) Tests d'activité herbicide comparés sur coton et xanthium

Le produit présente une bonne actiité herbicide sélective sur le coton qui n'est pas attaqué aux doses utilisées, alors que le xanthium est détruit totalement.

F) 1-(phénoxycarbonyl) 3-(2-tétrahydropyranyl) 5-éthyl-6-méthyl 1,2,3,4-tétrahydropyrimidine 2,4-dione
Test de post émergence

Dans ce test de post-émergence à 1,25 Kg/ha ou 0,625 Kg/ha, le maïs est inattaqué alors que les adventices sont détruites.

G) 1-(benzoxyl) 3-(2-tétrahydropyranyl) 5-éthyl 6-méthyl 1,2,3,4-tétrahydropyrimidine 2,4-dione
Test de pré-émergence

En test de pré-émergence, on note une action sélective, du composé test que attaque moins les graminés cultivées que les adventices. Cette action sélective est particulièrement nette sur le blé et aussi sur le maïs etr l'avoine.

H) 1-méthylsulfonyl) 3-(2-tétrahydropyranyl) 1,2,3,4,6,7-hexahydro 5H-cyclopentapyrimidine 2,4-dione
Test de pré-émergence

En test de pré-émergence, le composé testé permet de désherber sélectivement les graminés cultivées, notamment le blé et le maïs.

I) 1-(méthoxycarbonyl) 2-(2-tétrahydropyranyl) 1,2,3,4,6,7-hexahydro 5-H-cyclopentapyrimidine 2,4-dione

Le produit a été testé dans les conditions précédentes, et montré de très intéressantes propriétés herbicides, en particulier de remarquables propriétés herbicides sélectives sur coton qui n'est pas attaqué aux doses utilisées alors que le xanthium est détruit totalement.

**Revendications**

1. Les composés de formule générale (I)

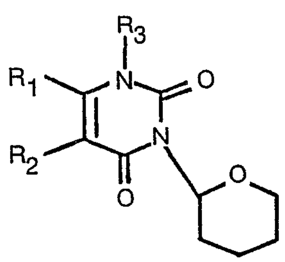

(I)

dans laquelle $R_1$ représente un radical alcoyle, linéaire ou ramifié, comportant de 1 à 3 atomes de carbone, $R_2$ représente un radical alcoyle linéaire ou ramifié comportant de 1 à 3 atomes de carbone, ou bien $R_1$ et $R_2$

représentent ensemble, avec les atomes de carbone auxquels ils sont liés, un homocycle carboné comportant de 5 à 7 atomes de carbone et $R_3$ représente *ou bien* un groupement

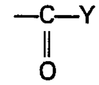

dans lequel Y représente un radical alcoyle, linéaire ou ramifié, comportant de 1 à 6 atomes de carbone, ou un radical phényle éventuellement substitué par un atome d'halogéne ou par un radical alcoyle ou alcoxy comportant de 1 à 3 atomes de carbone, *ou bien* un groupement $—CO_2Z$ dans lequel Z représente un radical alcoyle linéaire ou ramifié, comportant de 1 à 6 atomes de carbone, un radical cycloalcoyle comportant de 3 à 7 atomes de carbone, un radical phényle ou un radical benzyle, *ou bien* un groupement $SO_2Z_1$ dans lequel $Z_1$ représenté un radical alcoyle linéaire ou ramifié, comportant de 1 à 6 atomes de carbone.

2. Les composés de formule générale (I), tels que définis à la revendication 1, dans lesquels $R_1$ et $R_2$ représentent ensemble avec les carbones auxquels ils sont liés un homocycle carboné, comportant de 5 à 7 atomes de carbone et $R_3$ conserve les significations de la revendication 1.

3. Les composés de formule générale (I), tels que définis à la revendication 1, dans lesquels $R_1$ et $R_2$ représentent ensemble avec les carbones auxquels ils sont liés un homocycle carboné, comportant 5 atomes de carbone et $R_3$ représente un groupement $—CO_2Z$ dans lequel Z représente un radical alcoyle, linéaire ou ramifié, comportant de 1 à 3 atomes de carbone.

4. La 1-(méthoxycarbonyl) 3-(2-tétrahydropyranyl) 1,2,3,4,6,7-hexahydro 5H-cyclopentapyrimidine 2,4-dione.

5. Les composés de formule générale (I), tels que définis à la revendication 1, dans lesquels $R_1$ et $R_2$ représentent des radicaux alcoyles, linéaires ou ramifiés, comportant de 1 à 3 atomes de carbone et $R_3$ représente un groupement

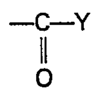

dans lequel Y représente un radical alcoyle, linéaire ou ramifié, comportant de 1 à 6 atomes de carbone ou un radical phényle.

6. Procédé de préparation des composés de formule générale (I) tels que définis à la revendication 1, caractérisé en ce que l'on fait réagir sur un composés de formule (II):

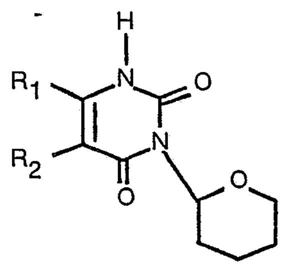

(II)

une base forte puis un composé de formule (III):

$$XR_3 \qquad \text{(III)}$$

dans laquelle X représente un halogène et de préférence le chlore et $R_3$ conserve les signifcations de la revendication 1.

7. Procédé de préparation selon la revendication 6 de la 1-(méthoxycarbonyl) 3-(2-tétrahydropyrranyl) 1,2,3,4,6,7-hexahydro 5H-cyclopentapyrimidine 2,4-dione, caractérisé en ce que l'on fait réagir sur la 3-(2-tétrahydropyranyl) 1,2,3,4,6,7-héxahydro 5H-cyclopentapyrimidine 2,4-dione, l'hydrure de sodium puis le chloroformiate de méthyle pour obtenir le produit recherché.

8. Les compositions pesticides notamment herbicides caractérisées en ce qu'elles contiennent, comme principe actif, un au moins des composés de formule générale (I) telle que définie à la revendication 1.

9. Les compositions pesticides, notamment herbicides, caractérisées en ce qu'elles contiennent, comme principe actif, un au moins des composés de formule générale (I) tesl que définis à l'une quelconque des revendications 2, 3 ou 5.

10. Les compositions pesticides, notamment herbicides, caractérisées en ce qu'elles contiennent, comme principe actif, le composé de la revendication 4.

11. Application des composés de formule (I), tels que définis à l'une quelconque des revendications 1, 2, 3, 4 ou 5 au désherbage sélectif des cultures de coton.

12. Application des composés de formule (I), tels que définis à l'une quelconque des revendications 1, 2, 3, 4 ou 5 au désherbage sélectif des cultures de soja.

13. Application des composés de formule (I), tels que définis à l'une quelconque des revendications 1, 2, 3, 4 ou 5 au déscherbage sélectif des cultures de blé, d'orge, de maïs et d'avoine.

**Patentansprüche**

1. Verbindung der allgemeinen Formel (I)

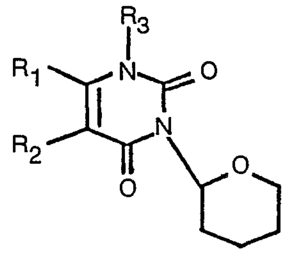

(I)

worin $R_1$ einen linearen oder verzweigten Alkylrest mit 1 bis 3 Kohlenstoffatomen bedeutet, $R_2$ einen linearen oder verzweigten Alkylrest mit 1 bis 3 Kohlenstoffatomen bedeutet oder $R_1$ und $R_2$ gemeinsam mit den Kohlenstoffatomen, an die sie gebunden sind, einen Kohlenstoff-Homocyclus mit 5 bis 7 Kohlenstoffatomen bilden und $R_3$ *entweder* eine Gruppe

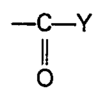

bedeutet, worin Y einen linearen oder verzweigten Alkylrest mit 1 bis 6 Kohlenstoffatomen oder einen gegebenenfalls durch ein Halogenatom oder einen Alkyl- oder Alkoxyrest mit 1 bis 3 Kohlenstoffatomen substituierten Phenylrest bedeutet, *oder* eine Gruppe $—CO_2Z$ darstellt, worin Z einen linearen oder verzweigten Alkylrest mit 1 bis 6 Kohlenstoffatomen, einen Cycloalkylrest mit 3 bis 7 Kohlenstoffatomen, einen Phenylrest oder einen Benzylrest bedeutet, *oder* eine Gruppe $SO_2Z_1$ bedeutet, worin $Z_1$ einen linearen oder verzweigten alkylrest mit 1 bis 6 Kohlenstoffatomen darstellt.

2. Verbindung der allgemeinen Formel (I) gemäß Anspruch 1, worin $R_1$ und $R_2$ gemeinsam mit den Kohlenstoffatomen, an die sie gebunden sind, einen Kohlenstoff-Homocyclus mit 5 bis 7 Kohlenstoffatomen bilden und $R_3$ die in Anspruch 1 angegebenen Bedeutungen besitzt.

3. Verbindung der allgemeinen Formel (I) gemäß Anspruch 1, worin $R_1$ und $R_2$ gemeinsam mit den Kohlenstoffatomen, an die sie gebunden sind, einen Kohlenstoff-Homocyclus mit 5 Kohlenstoffatomen bilden und $R_3$ eine Gruppe $—CO_2Z$ bedeutet, worin Z einen linearen oder verzweigten Alkylrest mit 1 bis 3 Kohlenstoffatomen darstellt.

4. 1-(Methoxycarbonyl)-3-(2-tetrahydropyranyl) 1,2,3,4,6,7-hexahydro-5H-cyclopentapyrimidin-2,4-dion.

5. Verbindungen der allgemeinen Formel (I) gemäß Anspruch 1, worin $R_1$ und $R_2$ lineare oder verzweigte Alkylreste mit 1 bis 3 Kohlenstoffatomen bedeuten und $R_3$ eine Gruppe

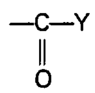

darstellt, worin Y einen linearen oder verzweigten Alkylrest mit 1 bis 6 Kohlenstoffatomen oder einen Phenylrest darstellt.

6. Verfahren zur Herstellung der Verbindungen der allgemeinen Formel (I) gemä7 Anspruch 1, dadurch gekennzeichnet, daß man mit einer Verbindung der Formel (II)

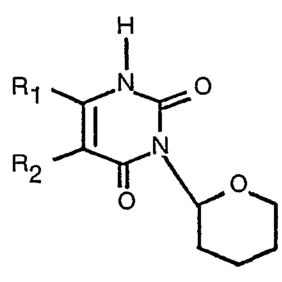

(II)

eine starke Base und danach eine Verbindung der Formel (III)

$$XR_3 \qquad (III)$$

umsetzt, worin X ein Halogenatom und vorzugsweise Chlor bedeutet und $R_3$ die in Anspruch 1 angegebenen Bedeutungen besitzt.

7. Verfahren gemä7 Anspruch 6 zur Herstellung von 1-(Methoxycarbonyl)-3-(2-tetrahydropyranyl)-1,2,3,4,6,7-hexahydro-5H-cyclopentapyrimidin-2,4-dion, dadurch gekennzeichnet, daß man mit 3-(Tetrahydropyranyl)-1,2,3,4,6,7-hexahydro-5H-cyclopentapyrimidin-2,4-dion Natriumhydrid und dann Methylchlorformiat umsetzt, um das gewünschte Produkt zu erhalten.

8. Pestizide, insbesondere herbizide Zusammensetzungen, dadurch gekennzeichnet, daß sie als Wirkstoff zumindest eine der Verbindungen der allgemeinen Formel (I) gemäß Anspruch 1 enthalten.

9. Pestizide, insbesondere herbizide Zusammensetzungen, dadurch gekennzeichnet, daß sie als Wirkstoff zumindest eine der Verbindungen der allgemeinen Formel (I) gemäß einem der Ansprüche 2, 3 oder 5 enthalten.

10. Pestizide, insbesondere herbizide Zusammensetzungen, dadurch gekennzeichnet, daß sie als Wirkstoff die Verbindung gemäß Anspruch 4 enthalten.

11. Verwendung der Verbindungen der Formel (I) gemäß einem der Ansprüche 1, 2, 3, 4, oder 5 zur selektiven Unkrautvernichtung von Baumwollkulturen.

12. Verwendung der Verbindungen der Formel (I) gemäß einem der Ansprüche 1, 2, 3, 4 oder 5 zur selektiven Unkrautvernichtung in Sojakulturen.

13. Verwendung der Verbindungen der Formel (I) gemäß einem der Ansprüche 1, 2, 3, 4 oder 5 zur selektiven Unkrautvernichtung in Kulturen von Weizen bzw. Getreide, Gerste, Mais und Hafer.

**Claims**

1. The compounds with the general formula (I):

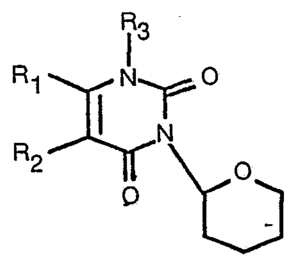

(I)

in which $R_1$ represents a linear or branched alkyl radical, including from 1 to 3 carbon atoms, $R_2$ represents a linear or branched alkyl radical, including from 1 to 3 carbon atoms, or $R_1$ and $R_2$, together with the carbon atoms to which they are attached, represent a carbon homocycle including from 5 to 6 carbon atoms and $R_3$ represents *either* a

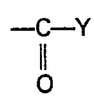

group in which Y represents a linear or branched alkyl radical, including from 1 to 6 carbon atoms, or a phenyl radical possibly substituted by a halogen atom or by an alkyl or an alkoxy radical including from 1 to 3 carbon atoms, *or* a $-CO_2Z$ group in which Z represents a linear or branched alkyl radical, including from 1 to 6 carbon atoms, a cycloalkyl radical including from 3 to 7 carbon atoms, a phenyl radical or a benzyl radical, *or* a $SO_2Z_1$ group in which $Z_1$ represents a linear or branched alkyl radical, including from 1 to 6 carbon atoms.

2. The compounds with the general formula (I), as defined in Claim 1, in which $R_1$ and $R_2$, together with the carbon atoms to which they are attached represent a carbon homocycle including from 5 to 7 carbon atoms and $R_3$ retains the significancies of Claim 1.

3. The compounds with the general formula (I), as defined in Claim 1, in which $R_1$ and $R_2$, together with the carbon atoms to which they are attached, represent a carbon homocycle including 5 carbon atoms, and $R_3$ represents a $-CO_2Z$ group in which Z represents a linear or branched alkyl radical including from 1 to 3 carbon atoms.

4. 1-(methoxycarbonyl)-3-(2-tetrahydropyranyl)-1,2,3,4,6,7-hexahydro-5H-cyclopentapyrimidine-2,4-dione.

5. The compounds with the general formula (I), as defined in Claim 1, in which $R_1$ and $R_2$ represent linear or branched alkyl radicals, including from 1 to 3 carbon atoms and $R_3$ represents a

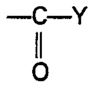

group in which Y represents a linear or branched alkyl radical, including from 1 to 6 carbon atoms, or a phenyl radical.

6. Preparation process for the compounds with the general formula (I), as defined in Claim 1, characterized in that a strong base and then a compound with the formula (III):

$$XR_3 \qquad (III)$$

in which X represents a halogen and preferably chlorine and $R_3$ retains the significancies of Claim 1, is made to react on a compound with the formula (II):

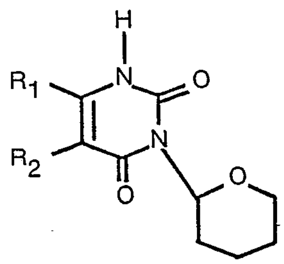

(II)

7. Preparation process according to Claim 6 of 1-(methoxycarbonyl)-3-(2-tetrahydropyranyl)-1,2,3,4,6,7-hexahydro-5H-cyclopentapyrimidine-2,4-dione, characterized in that sodium hydride and then methyl chloroformate are made to react on 3-(2-tetrahydropyranyl)-1,2,3,4,6,7-hexahydro-5H-cyclopentapyrimidine-2,4-dione in order to obtain the product sought.

8. Pesticide and in particular herbicide compositions, characterized in that they contain as active principle, at least one of the compounds with the general formula (I) as defined in Claim 1.

9. Pesticide and in particular herbicide compositions, characterized in that they contain as active principle, at least one of the compounds with the general formula (I) as defined in any one of the Claims 2, 3 or 5.

10. Pesticide and in particular herbicide compositions, characterized in that they contain, as active principle the compound of Claim 4.

11. Use of the compounds with the formula (I), as defined in any one of the Claims 1, 2, 3, 4 or 5 for selective weeding of cotton cultures.

12. Use of the compounds with the formula (I), as defined in any one of the Claims 1, 2, 3, 4 or 5 for selective weeding of soya cultures.

13. Use of the compounds with the formula (I), as defined in any one of the Claims 1, 2, 3, 4 or 5 for selective weeding of wheat, barley, maize and oat cultures.